(19) Europäisches Patentamt

European Patent Office

Office européen des brevets

(11) **EP 1 098 875 B1**

(12) **FASCICULE DE BREVET EUROPEEN**

(45) Date de publication et mention
de la délivrance du brevet:
**04.02.2004 Bulletin 2004/06**

(51) Int Cl.[7]: **C07D 201/08**

(21) Numéro de dépôt: **99931348.9**

(86) Numéro de dépôt international:
**PCT/FR1999/001730**

(22) Date de dépôt: **15.07.1999**

(87) Numéro de publication internationale:
**WO 2000/005203 (03.02.2000 Gazette 2000/05)**

(54) **PROCEDE D'HYDROLYSE CYCLISANTE D'UN COMPOSE AMINONITRILE EN LACTAME**

VERFAHREN ZUR HERSTELLUNG VON LACTAMEN DURCH CYCLISIERENDE HYDROLYSE
VON AMINONITRILEN

METHOD FOR CYCLIZING HYDROLYSIS OF AN AMINONITRILE COMPOUND INTO LACTAM

(84) Etats contractants désignés:
**AT BE CH CY DE DK ES FI FR GB GR IE IT LI LU
MC NL PT SE**

(30) Priorité: **22.07.1998 FR 9809529**

(43) Date de publication de la demande:
**16.05.2001 Bulletin 2001/20**

(73) Titulaire: **Rhodia Polyamide Intermediates
69192 Saint-Fons (FR)**

(72) Inventeurs:
• **BRUNELLE, Jean-Pierre
78290 Croissy-sur-Seine (FR)**
• **SEIGNEURIN, Aline
78150 Le Chesnay (FR)**
• **SEVER, Lionel
69007 Lyon (FR)**

(74) Mandataire: **Esson, Jean-Pierre et al
Rhodia Services,
Direction de la Propriété Industrielle,
Centre de Recherches de Lyon
BP 62
69192 Saint-Fons Cédex (FR)**

(56) Documents cités:
**EP-A- 0 748 797       WO-A-96/22974
DE-A- 19 632 006**

Remarques:
Le dossier contient des informations techniques
présentées postérieurement au dépôt de la
demande et ne figurant pas dans le présent
fascicule.

**Description**

L'invention concerne un procédé d'hydrolyse cyclisante d'un composé aminonitrile en lactame en présence d'un catalyseur.

Elle se rapporte plus particulièrement à un procédé d'hydrolyse cyclisante d'un composé aminonitrile en présence d'un catalyseur particulaire macroporeux obtenu par mise en forme d'une poudre d'au moins un oxyde minéral.

Les lactames, tels l'epsilon-caprolactame, sont des composés de base pour la fabrication de polyamides et notamment de PA 6.

Parmi les différents procédés connus de synthèse de ces lactames, un des procédés est l'hydrolyse cyclisante de l'aminonitrile correspondant, et plus particulièrement d'un aminonitrile aliphatique correspondant, en présence d'eau et d'un catalyseur.

Ainsi, le brevet US 2,357,484 décrit un procédé de préparation en phase vapeur de lactame, avec comme catalyseur de l'alumine activée, de la silice sous forme de gel ou de l'acide borophosphorique.

Le brevet US 4,628,085 décrit également un procédé de préparation de lactames par hydrolyse cyclisante en phase vapeur en présence d'un catalyseur à base de silice, de surface spécifique supérieure à 250 $m^2$/g. Cette réaction est réalisée en présence d'hydrogène et d'ammoniac.

La demande WO 98/0669 propose des catalyseurs à base d'oxydes métalliques hydratés ou non, les métaux étant choisis dans le groupe comprenant l'étain, le zirconium, l'hafnium, le bismuth, le vanadium, le niobium, le tantale ou leurs mélanges. Ces catalyseurs sont du type massique et ne présentent pas de macroporosité. Leur durée de cycle est très courte et incompatible avec une exploitation industrielle du procédé de fabrication de lactame. De même, le brevet DE19632006 décrit l'utilisation d'oxydes métalliques comme catalyseur d'hydrlyse cyclisante soit sous forme massique soit sous forme supportée, par exemple sur un support alumine. Toutefois, les taux de transformation et la sélectivité en lactames obtenus sont trop faibles pour permettre la production d'un lactame de qualité acceptable pour les applications polymères, notamment dans le domaine du filage.

Le brevet EP 748797 décrit l'utilisation de zéolithes comme catalyseur de l'hydrolyse cyclisante d'un aminonitrile. Toutefois, le taux de tranformation de l'aminonitrile est d'un niveau peu élevé par rapport à d'autres catalyseurs tels que ceux décrits dans le document ci-dessous

En outre, la Demanderesse a proposé dans la demande de brevet WO96/22974, un procédé d'hydrolyse cyclisante d'aminonitrile en phase vapeur utilisant comme catalyseur une alumine présentant des domaines de surface spécifique et de volume poreux déterminés.

Les lactames produits sont généralement utilisés pour la fabrication de polymère, comme par exemple l'epsilon-caprolactame pour la fabrication du PA 6. Les applicaticns de ces polymères sont variées et nombreuses.

Toutefois, une des plus importantes est la fabrication de fils, filaments ou fibres pour notamment l'industrie textile.

Ces applications variées et notamment celle décrite ci-dessus requièrent l'utilisation d'un polyamide présentant des caractéristiques physico-chimiques et chimiques très précises. Pour obtenir de telles caractéristiques, il est nécessaire de synthétiser ces polymères à partir de monomères ou lactames présentant également des caractéristiques de pureté très sévères.

Ainsi, le caprolactame doit généralement respecter les spécifications suivantes :

- indice de permanganate (selon norme ISO 8660) : < 5
- bases libres : < 0,1 milliéquivalent (meq)/kg de CPL
- bases volatiles (selon norme ISO 8661) : < 0,5 meq/kg
- absorbance UV à 290 nm (selon norme ISO 7059) : < 0,05

Pour obtenir celles-ci, il est nécessaire de mettre en oeuvre des procédés complexes de purification. De tels procédés présentent de nombreux inconvénients d'ordre économique notamment une consommation énergétique et des investissements en matériels importants.

Une des causes de la nécessité de purifier le lactame brut produit par les procédés connus est la présence de réactions secondaires qui interviennent notamment pendant l'étape d'hydrolyse cyclisante de l'aminonitrile.

Pour éviter ces réactions secondaires, il faut donc que le catalyseur favorise la réaction principale de formation du lactame. Cette caractéristique du catalyseur peut être illustrée par la sélectivité du procédé en lactame brut. On peut également apprécier la qualité d'un lactame brut par titrage de ce dernier avec une solution aqueuse de permanganate de potassium 0,2 N (indice de permanganate).

En outre, pour que la pureté du lactame brut produit soit élevée et continue, il est nécessaire que le niveau de sélectivité du catalyseur soit conservé pendant toute la durée de cycle de celui-ci.

Les alumines proposées dans la demande de brevet de la Demanderesse WO96/22974 représente un début de solution à ce problème en proposant un catalyseur à activité et sélectivité élevées avec une durée de cycle du catalyseur élevée.

Toutefois, il apparaît nécessaire d'améliorer encore les performances de la catalyse de l'hydrolyse cyclisante des lactames, pour améliorer la sélectivité initiale du catalyseur ainsi que la pureté du lactame brut, tout en conservant ce niveau élevé pendant toute la durée de cycle du catalyseur.

Un des buts de la présente invention est de proposer une solution pour améliorer la catalyse de l'hydrolyse cyclisante des aminonitriles, et notamment la sélectivité de cette catalyse pour produire un lactame brut de degré de pureté élevée. Ainsi, le lactame brut produit peut être utilisé pour la fabrication de polymère de caractéristiques chimiques et physico-chimiques élevées, après mise en oeuvre d'un procédé de purification plus simple et plus économique que ceux mis en oeuvre actuellement.

Par lactame brut, on entend le produit issu de la réaction d'hydrolyse cyclisante après élimination de l'ammoniac et des solvants éventuels tels que l'eau, par exemple.

Par lactame purifié, on désigne le lactame obtenu par purification du lactame brut.

A cet effet, l'invention propose un procédé d'hydrolyse cyclisante d'un composé aminonitrile en lactame par réaction d'un aminonitrile de formule générale (I) suivante :

$$N \equiv C - R - NH_2 \qquad \qquad (I)$$

dans laquelle :

R représente un radical aliphatique, cycloaliphatique, arylaliphatique substitué ou non comprenant de 3 à 12 atomes de carbone, avec de l'eau, en présence d'un catalyseur solide, caractérisé en ce que le catalyseur est un catalyseur particulaire obtenu par dépôt et/ou adsorption d'au moins un composé oxygéné d'au moins un élément choisi dans le groupe consistant en les éléments appartenant aux groupes 1 à 16 de la classification universelle des éléments ( nouvelle classification), cette liste incluant également les terres rares, sur un support particulaire en oxyde minéral simple ou mixte d'au moins un élément choisi dans le groupe consistant en le silicium, l'aluminium, le titane, le zirconium, le vanadium, le niobium, le tantale, le tungstène, le molybdène, le fer, les terres rares, ou par mélange d'au moins un desdits composés oxygénés ou un précurseur desdits composés oxygénés avec le ou les oxydes minéraux formant le support avant leur mise en forme.

Selon l'invention, le catalyseur particulaire comprend au moins une macroporosité caractérisé par un volume poreux correspondant aux pores de diamètre supérieur à 500 Å, supérieur ou égal à 5ml/100g.

Cette macroporosité est formée avantageusement pendant le procédé de mise en forme des particules par des techniques décrites ci-dessous, ou comme par exemple l'addition de porogène.

Le catalyseur peut être mis en oeuvre sous diverses formes telles que billes, concassés, extrudés en forme de granulés cylindrique creux ou pleins, de nid d'abeille, pastilles, la mise en forme pouvant éventuellement être réalisée à l'aide d'un liant.

Il peut s'agir tout d'abord de billes d'oxydes minéraux issues d'une mise en forme par oil-drop (ou coagulation en gouttes). Ce type de billes peut par exemple être préparé par un procédé similaire à celui décrit pour la formation de billes d'alumine dans les brevets EP-A-0 015 801 ou EP-A-0 097 539. Le contrôle de la porosité peut être réalisé en particulier, selon le procédé décrit dans le brevet EP-A-0 097 539, par coagulation en gouttes d'une suspension ou d'une dispersion aqueuse d'oxyde minéral.

Les billes peuvent être également obtenues par un procédé d'agglomération dans un drageoir ou tambour tournant.

Il peut aussi s'agir d'extrudés d'oxydes minéraux. Ceux-ci peuvent être obtenus par malaxage, puis extrusion d'une matière à base de l'oxyde minéral. Le contrôle de la porosité de ces extrudés peut être réalisé par le choix de l'oxyde mis en oeuvre et par les conditions de préparation de cet oxyde ou par les conditions de malaxage de cet oxyde avant extrusion. L'oxyde minéral peut ainsi être mélangé lors du malaxage à des porogènes. A titre d'exemple, les extrudés peuvent être préparés par le procédé décrit dans le brevet US 3 856 708.

De manière semblable, des billes à porosité contrôlée peuvent être obtenues par addition de porogène et agglomération dans un bol tournant ou drageoir ou par le procédé 'Oil-drop'.

Ces procédés de mise en forme peuvent être effectués par mise en oeuvre d'un mélange de l'oxyde minéral et d'un composé oxydé conforme à l'invention, ou d'un précurseur dudit composé oxygéné, le précurseur étant transformé en composé oxydé par traitement thermique du catalyseur par exemple et avantageusement après mise en forme.

Selon une autre caractéristique de l'invention, les particules de catalyseur présente une surface spécifique supérieure à 10 m$^2$/g et un volume poreux égal ou supérieur à 10 ml/100 g, le volume poreux correspondant aux pores de diamètre supérieur à 500 Å étant supérieur ou égal à 10 ml/100 g.

Selon une autre caractéristique de l'invention, les particules de catalyseur présentent une surface spécifique supérieure à 50 m$^2$/g.

Avantageusement, elles présentent un volume poreux total supérieur ou égal à 15 ml/100g avec un volume poreux correspondant aux pores de diamètre supérieur à 200 Å supérieur ou égal à 15 ml/100g, de préférence supérieur ou égal à 20ml/100g.

Selon une autre caractéristique de l'invention, le catalyseur présente un volume poreux total supérieur ou égal à 20 ml/100g avec un volume poreux correspondant aux pores de diamètre supérieur à 70 Å supérieur ou égal à 20 ml/ 100g.

Dans le mode opératoire comprenant un support poreux supportant des composés oxygénés d'éléments, ces éléments sont avantageusement choisis dans la liste comprenant le silicium, le titane, le zirconium, le vanadium, le niobium, le tantale, le tungstène, le molybdène, le phosphore, le bore, le fer, les alcalino- terreux, les terres rares. Le composé oxygéné est avantageusement un oxyde simple ou mixte de un ou plusieurs des éléments cités ci-dessus ou un mélange de ces oxydes.

Dans ce mode de réalisation, le support poreux est de préférence une alumine poreuse. Avantageusement, cette alumine présente les caractéristiques de surface spécifique, volume poreux et distribution de pores définis précédemment.

Ce mode de réalisation est particulièrement avantageux car la porosité spécifique de ce catalyseur présente dans la catalyse de la réaction d'hydrolyse cyclisante des aminonitriles, une durée de cycle élevée comme cela est décrit dans la demande de brevet WO 96/22974. La présence des composés oxygénés cités ci-dessus à la surface des pores permet d'avoir un effet catalytique amélioré par rapport au catalyseur réalisé avec le même composé oxygéné mais sous forme massique et non supporté. Dans le cas où l'oxyde minéral formant le support a une activité catalytique dans la réaction d'hydrolyse cyclisante, la présence de ces composés oxydés modifient l'activité catalytique du catalyseur en améliorant notamment la sélectivité de la réaction, en minimisant les réactions secondaires.

Selon une autre caractéristique de l'invention, l'efficacité du catalyseur et plus particulièrement sa sélectivité peuvent être améliorées par la présence avec le composé oxygéné d'anions choisis dans le groupe comprenant le fluor, des anions de formule générale (MxOy) dans la quelle M représente un élément choisi dans le groupe comprenant le silicium, l'arsenic, l'antimoine, l'azote, le soufre, le carbone et le phosphore, x étant un nombre entier compris entre 1 et 4 et y un nombre entier compris entre 1 et 8, ou des hétéropolyanions (HPA) de formule générale $X^{(n)+}T_{12}O_{40}^{(8-n)-}$ dans laquelle T signifie le tungstène ou le molybdène et X signifie le silicium, le germanium, le phosphore, l'arsenic ou le vanadium.

A titre d'exemple d'anions plus particulièrement convenables pour l'invention; on peut notamment citer les phosphates, sulfates, silicates, et les hétéropolyanions dodécamolybdates et dodécatungstates.

En conséquence le lactame brut obtenu est de meilleure qualité. L'obtention d'un lactame purifié satisfaisant les spécifications indiqués précédemment est facilitée.

La concentration pondérale en composé oxygéné présent dans le catalyseur est avantageusement comprise entre 1000 ppm et 30% exprimée en masse d'élément du composé oxygéné par rapport à la masse totale du catalyseur. Cette concentration est plus préférentiellement comprise entre 0,5 % et 15 % en poids.

La concentration en anions présents dans le catalyseur est avantageusement comprise entre 0,5 et 15% en poids.

Quand les supports poreux correspondent à des alumines conformes à l'invention, celles-ci sont obtenues généralement par déshydratation de gibbsite, bayerite, nordstrandite ou de leurs différents mélanges. Les différents procédés de préparation des alumines sont décrits dans l'encyclopédie KIRK-OTHMER, volume 2, pages 291 - 297.

On peut préparer les alumines mises en oeuvre dans le présent procédé par mise en contact d'une alumine hydratée, sous forme finement divisée, avec un courant de gaz chaud à une température comprise entre 400°C et 1000°C, puis maintien du contact entre l'hydrate et les gaz pendant une durée allant d'une fraction de seconde jusqu'à 10 secondes et enfin séparation de l'alumine partiellement déshydratée et des gaz chauds. On peut notamment se référer au procédé décrit dans le brevet américain US 2 915 365.

On peut également procéder à l'autoclavage d'agglomérés d'alumines obtenues précédemment, en milieu aqueux, éventuellement en présence d'acide, à une température supérieure à 100°C et de préférence comprise entre 150°C et 250°C, pendant une durée de préférence comprise entre 1 et 20 heures, puis à leur séchage et leur calcination.

La température de calcination est réglée de telle façon que l'on obtienne des surfaces spécifiques et des volumes poreux situés dans les zones de valeurs indiquées précédemment.

Les catalyseurs de l'invention ont avantageusement une surface spécifique supérieure à 50 m$^2$/g.

En outre, ils présentent avantageusement des pores de diamètre supérieur à 0,1μm, le volume poreux apportés par ces pores étant supérieur ou égal à 5ml/100g, avantageusement supérieur ou égal à 10ml/100g.

Dans un mode de réalisation préféré de l'invention, ces catalyseurs comprennent également des pores de diamètre égal ou supérieur à 0,5 μm, le volume poreux correspondant étant égal ou supérieur à 5 ml/100 g, de préférence supérieur ou égal à 10 ml/100 g.

Ce volume poreux généré par les pores de diamètre supérieur à 500 Å, de préférence supérieur à 0,1 μm et avantageusement supérieur à 0,5 μm permet d'obtenir des catalyseurs à durée de cycle élevée en tant que catalyseur de la réaction d'hydrolyse cyclisante des aminonitriles en lactames. Ainsi, de tels catalyseurs peuvent être utilisés dans des procédés industriels de production de lactames.

Selon l'invention, les catalyseurs comprenant des composés oxygénés supportés par un support poreux sont obtenus, généralement par imprégnation du support, notamment de l'alumine, par une solution d'un sel ou composés des éléments cités précédemment, puis séchés et calcinés à une température égale ou supérieure à 400°C, pour transformer éventuellement et avantageusement lesdits composés ou sels en composés oxygénés, de préférence en oxydes.

De même l'addition des anions peut être réalisée par mise en contact du support poreux avant imprégnation par les composés oxygénés ou conjointement à cette imprégnation avec une solution contenant des sels à base de ces anions avantageusement décomposables thermiquement tels que les sels d'ammonium. Cette addition peut être également réalisée par mise en contact du catalyseur poreux comprenant le composé oxygéné avec une solution contenant l'anion à ajouter.

Les oxydes et les anions sont par ces modes de réalisation généralement présents à la surface des pores du support.

Dans un autre mode de réalisation déjà évoqué précédemment, les composés d'éléments peuvent être ajoutés dans le matériau constituant le support avant sa mise en forme ou au cours du processus de mise en forme.

La calcination des supports imprégnés est de préférence réalisée sous atmosphère oxydante telle que l'air.

Ainsi, comme cela sera démontré ci-après, la réaction d'hydrolyse cyclisante peut être réalisée avec un minimum de réactions secondaires, améliorant de manière sensible la sélectivité du procédé en lactame et donc la pureté du produit brut obtenu.

La réaction d'hydrolyse cyclisante nécessite la présence d'eau. Le rapport molaire entre l'eau et l'aminonitrile engagés se situe habituellement entre 0,5 et 50 et de préférence entre 1 et 20. La valeur supérieure de ce rapport n'est pas critique pour l'invention, mais des rapports plus élevés n'ont guère d'intérêt pour des questions économiques.

L'aminonitrile et l'eau peuvent être engagés sous forme de leurs mélanges à l'état de vapeur.

Dans un autre mode de réalisation, les réactifs aminonitrile et eau sont engagés à l'état liquide sous pression éventuellement en présence d'un solvant.

Dans le mode préféré de l'invention, les réactifs sont maintenus à l'état de vapeur dans le réacteur chargé avec une quantité déterminée de catalyseur.

Le volume libre du réacteur peut être occupé par un solide inerte tel que, par exemple, du quartz, afin de favoriser la vaporisation et la dispersion des réactifs.

On peut sans inconvénient utiliser tout gaz inerte comme vecteur, tel que l'azote, l'hélium ou l'argon.

La température à laquelle est mis en oeuvre le procédé de l'invention doit être suffisante pour que les réactifs soient bien à l'état de vapeur. Elle se situe généralement entre 200°C et 450°C et de préférence entre 250°C et 400°C.

Le temps de contact entre l'aminonitrile et le catalyseur n'est pas critique. Ce temps de contact se situe de préférence entre 0,5 à 200 secondes et encore plus préférentiellement entre 1 et 100 secondes.

La pression n'est pas un paramètre critique du procédé. Ainsi on peut opérer sous des pressions de $10^{-3}$ bar à 200 bar. De préférence, on mettra en oeuvre le procédé sous une pression de 0,1 à 20 bar. Dans le cas d'une hydrolyse réalisée en phase vapeur, cette pression est avantageusement comprise entre $10^{-3}$ bar et 3 bar.

Il n'est pas exclu d'utiliser un solvant inerte dans les conditions réactionnelles, tel que par exemple un alcane, un cycloatcane, un hydrocarbure aromatique ou l'un de ces hydrocarbures précédents halogéné, et d'avoir ainsi une phase liquide dans le flux réactionnel.

Les aminonitriles qui peuvent être cyclisés par le procédé de l'invention sont avantageusement les ω-aminonitriles aliphatiques tel que l'ω-aminovaleronitrile, ω-aminocapronitrile, ω-aminooctanitrile, ω-aminononanitrile, ω-aminodécanitrile, ω-aminodécanonitrile, ω-aminododécanonitrile, méthyl-aminovaléronitrile.

Le composé préféré et le plus important est l'aminocapronitrile qui conduit à l'ε-caprolactame. Ce dernier composé est le monomère du polyamide 6 utilisé pour la fabrication de différents articles tels que pièces moulées, fils, fibres, filaments, câbles ou films.

L'ε-caprolactame produit par la réaction d'hydrolyse cyclisante est avantageusement purifié par les différents procédés connus de purification, tels que distillation, cristallisation en milieu solvant ou en phase fondue, traitement sur résine, traitement par un oxydant et/ou hydrogénation. Ces différentes étapes peuvent être partiellement ou totalement combinées dans des ordres différents et selon le degré de pureté de l'ε-caprolactame produit.

Un des avantages de l'invention réside dans la simplification du procédé de purification en rie mettant en oeuvre qu'une ou deux de ces étapes.

D'autres buts, avantages et détails apparaîtront plus clairement au vu des exemples donnés ci-dessous uniquement à titre indicatif.

## EXEMPLES 1 A 3

Dans un réacteur cylindrique de 40 mm de diamètre et de hauteur égale à 1 m, 200 g de catalyseur sont chargés et répartis dans le réacteur de la manière suivante :

- dans un premier tronçon de réacteur 43,7 g de catalyseur sont mélangés avec 889 g de billes de verre
- dans un second tronçon du réacteur 156,3 g de catalyseur sont mélangés avec 169 g de billes de verre

De l'eau et de l'aminocapronitrile sont injectés selon des débits massiques égaux respectivement à 129 g/h et 200 g/h.

Le réacteur est maintenu à une température de 300°C.

L'essai est arrêté après une durée de fonctionnement de 500 heures.

Le mélange réactionnel est dosé en chromatographie en phase vapeur, notamment pour déterminer la concentration en caprolactame.

On détermine le taux de transformation (TT) de l'aminocapronitrile et la sélectivité S en caprolactame (CPL) par rapport à l'aminocapronitrile transformé.

La qualité du caprolactame brut obtenu est mesurée par titration d'une solution sulfurique de caprolactame par une solution aqueuse de permanganate de potassium 0,2 N. Elle s'exprime en ml de solution de $KMnO_4$ par kg de caprolactame.

Ce dosage est réalisé selon le mode opératoire suivant :

3g d'une solution contenant le caprolactame dont la concentration en caprolactame a été déterminée par chromatographie en phase liquide, sont ajoutés dans 60ml d'eau. 3 ml d'une solution d'acide sulfurique concentrée (98%) sont ajoutés dans la solution de caprolactame. La mesure de l'indice permanganate déterminé après neutralisation du milieu à un pH de 7 est réalisée par addition d'une solution de permanganate de potassium 0,2N.

L'alumine utilisée comme catalyseur présente les caractéristiques suivantes :

- <u>Alumine</u> :

  - surface spécifique (SS) : 139 $m^2$/g
  - Volume poreux total: 117ml/100g
  - Volume poreux correspondant aux pores de diamètre supérieur à 500 Å : 50ml/100g
  - Volume poreux correspondant aux pores de diamètre supérieur à 200 Å : 70ml/100g
  - Volume poreux correspondant aux pores de diamètre supérieur à 70 Å : 116ml/100g.

Le tableau ci-dessous rassemble les résultats obtenus dans :

- l'exemple 1 qui est représentatif de l'art antérieur (demande de brevet WO96/22974) et a été réalisé avec l'alumine décrite ci-dessus ;
- les exemples 2 et 3 sont représentatifs de l'invention ; ils ont été réalisés avec des catalyseurs constitués respectivement par imprégnation, séchage et calcination de 3 % de $TiO_2$ et 3 % de $La_2O_3$ déposés sur l'alumine décrite précédemment.

| Exemples | | | 1 | 2 | 3 |
|---|---|---|---|---|---|
| Catalyseur | | | Alumine | alumine + 3% TiO$_2$ | alumine + 3% La$_2$O$_3$ |
| Après 500 heures | | TT en ACN | 96,3 % | 97,1 | 96,5 |
| | | S en CPL | >99 % | >99 % | >99 % |
| | | qualité du CPL brut (ml KMnO4 / Kg CPL) | 123 | 62 | 91 |

### Tableau I

### EXEMPLES 4 A 11

Dans un réacteur cylindrique de 40 mm de diamètre et de hauteur égale à 1 m, 166,5 g de catalyseur sont chargés et répartis dans le réacteur de la manière suivante :

- dans un premier tronçon de réacteur 36,5 g de catalyseur sont mélangés avec 845 g de billes de verre
- dans un second tronçon du réacteur 130 g de catalyseur sans billes de verre

De l'eau et de l'aminocapronitrile sont injectés selon des débits massiques égaux respectivement à 128 g/h et 200 g/h.

Le réacteur est maintenu à une température de 300°C.

L'essai est arrêté après une durée de fonctionnement de 200 heures.

Le mélange réactionnel est dosé en chromatographie en phase vapeur, notamment pour déterminer la concentration en caprolactame.

On détermine le taux de transformation (TT) de l'aminocapronitrile et la sélectivité S en caprolactame (CPL) par rapport à l'aminocapronitrile transformé.

La qualité du caprolactame brut contenu dans le milieu réactionnel est appréciée par mesure de l'indice permanganate déterminé par neutralisation du milieu à un pH de 7 par addition d'une solution d'acide chlorhydrique et mesure de l'absorbance UV à une longueur d'onde de 290nm. Cette qualité est également caractérisée par la détermination de l'indice polarographique appelé IPOL qui est représentatif de la concentration en imines électroréductibles dans le milieu analysé.

Cet indice est déterminé selon la procédure suivante :

L'électrolyte (une solution à 10% en poids de HMD dans de l'eau) est introduit dans un vase polarographique de volume 10 ou 20 ml. Après désaération sous agitation, l'électrolyte est soumis à un flux d'argon ou d'azote pendant 5 minutes.

Un polarogramme "blanc" est réalisé sous atmosphère d'azote ou d'argon. Dans cet électrolyte, 0,1g d'échantillon à analyser sont ajoutés. Après désaération et passage sous atmosphère inerte d'azote ou d'argon, un polarogramme "blanc+echantillon" est établi.

Un échantillon étalon isobutanal contenant 500mg d'isobutanal dans du méthanol, le volume de solution étant de 50 ml, est ajouté dans le milieu électrolyte plus échantillon. Un polarogramme est établi dans les mêmes conditions que ci-dessus.

Les polarogrammes sont relevés dans les conditions suivantes :

- Potentiel initial = -1,1 V / Ag-AgCl
- Potentiel final = -1,9 V /Ag-AgCl
- Vitesse de balayage = 4 mV / s

Le calcul de l'indice IPOL est donné par la formule :

$$IPOL = \frac{10^6}{72{,}11} \times \frac{V \times U}{M^1} \times \frac{I_2 - I_1}{I_3 - I_2}$$

dans laquelle :

- M' représente la masse en gramme de la prise d'échantillon du milieu à analyser
- $I_1$ représente le courant de réduction correspondant au "blanc" d'électrolyte en nA, mesuré à -1,7 V / Ag - AgCl
- $I_2$ représente le courant de réduction correspondant au "blanc + échantilion",, mesuré à -1,7 V / Ag - AgCl
- $I_3$ représente le courant de réduction correspondant au "blanc + échantillon + etalon",, mesuré à -1.7 V / Ag - AgCl
- V représente en ml le volume de l'ajout de l'étalon

  U représente le titre en g/l d'isobutanal dans la solution étalon
  IPOL est l'Indice Polarographique des imines exprimés en mmole d'isobutanal/tonne d'échantillon.

L' alumine utilisée comme catalyseur présente les caractéristiques suivantes :

- surface spécifique (SS) : 140 $m^2$/g
- Volume poreux total: 112,9 ml/100g
- Volume poreux correspondant aux pores de diamètre supérieur à 10000 Å : 12 ml/100g
- Volume poreux correspondant aux pores de diamètre supérieur à 1000 Å : 34,8 ml/100g
- Volume poreux correspondant aux pores de diamètre supérieur à 200 Å : 92 ml/100g
- Volume poreux correspondant aux pores de diamètre supérieur à 70 Å : 111 ml/100g.

Le tableau II ci-dessous rassemble les résultats obtenus.

- l'exemple 4 comparatif a été réalisé avec comme catalyseur l'alumine ci-dessus exempt de composés oxydés et/ou d'anions
- les exemples 5 à 11 sont représentatifs de l'invention. Ils ont été réalisés avec des catalyseurs obtenus par imprégnation de l'alumine précitée par un précurseur d'éléments oxygénés, séchage et calcination. La composition des catalyseurs testés est indiquée dans le tableau II ci-dessous.

Ce tableau II rassemble également les résultats obtenus sur l'activité du catalyseur en terme de taux de transformation de l'ACN et sélectivité en caprolactame.
Les résultats sur la qualité du caprolactame obtenu sont rassemblés dans le tableau III. Ces résultats montrent clairement les effets de la présence de ces éléments oxygénés à la surface du support poreux aussi bien sur la teneur en imines ( indice IPOL) que sur l'indice permanganate et absorbance UV.
En conséquence, l'invention permet de produire un caprolactame plus pur qui pourra être purifié plus facilement et avec moins d'étapes élémentaires de purification pour respecter les spécifications requises pour son utilisation, notamment pour la fabrication de polyamide à application textile.

Tableau II

| N° exempte | Catalyseur (% molaire de l'élément par rapport au poids du catalyseur) | TT (ACN) en % | S (CPL) en % |
|---|---|---|---|
| 4 | $Al_2O_3$ | 99,1 | 99, 8 |
| 5 | $Al_2O_3$ , Fe(3%) | 99,4 | 99,9 |
| 6 | $Al_2O_3$ ,P (3%) | 99,4 | 99,9 |
| 7 | $Al_2O_3$ , Ti (3%) | 99,6 | 99,9 |
| 8 | $Al_2O_3$ , Ti (8%) | 99,7 | 99,5 |
| 9 | $Al_2O_3$ , Sn (3%) | 99,7 | 99,7 |
| 10 | $Al_2O_3$ , V(3%) | 99,5 | 99,9 |
| 11 | $Al_2O_3$, W (3%) | 99,4 | 100 |
| 12 | $Al_2O_3$ , Ti (1,5%), Fe (1,5%) | 99,7 | 100 |

Tableau III

| N° exemple | IPOL | Indice KMnO$_4$ | Absorbance à 290nm |
|---|---|---|---|
| 4 | 4200 | 409 | 1,9 |
| 5 | 990 | 235 | 1,0 |
| 6 | 1105 | 299 | 1,4 |
| 7 | 2425 | 271 | 1,5 |
| 8 | 2235 | 172 | 2,0* |
| 9 | 3133 | 238 | 1,65 |
| 10 | 1545 | 251 | 1,5 |
| 11 | 715 | 293 | 1,2 |
| 12 | 1860 | 111 | 0,9 |

**Revendications**

1. Procédé d'hydrolyse cyclisante d'un composé aminonitrile en lactame par réaction d'un aminonitrile de formule générale (I) :

$$N \equiv C - R - NH_2 \tag{I}$$

dans laquelle R représente un radical aliphatique, cycloaliphatique, arylaliphatique substitué ou non comprenant de 3 à 12 atomes de carbone, avec de l'eau, en présence d'un catalyseur solide, **caractérisé en ce que** le catalyseur est un catalyseur particulaire obtenu par par dépôt et/ou adsorption d'au moins un composé oxygéné d'au moins un élément choisi dans le groupe consistant en les éléments appartenant aux groupes 1 à 16 de la classification universelle des éléments ( nouvelle classification), cette liste incluant également les terres rares, sur un support en oxyde minéral simple ou mixte ou mélange d'oxydes d'au moins un élément choisi dans le groupe consistant en le silicium, l'aluminium, le titane, le zirconium, le vanadium, le niobium, le tantale, le tungstène, le molybdène, le fer, les terres rares, et **en ce qu'**il comprend au moins une macroporosité **caractérisé par** un volume poreux correspondant aux pores de diamètre supérieur à 500 Å, supérieur ou égal à 5ml/100g.

2. Procédé selon la revendication 1, **caractérisé en ce que** le catalyseur particulaire présente une surface spécifique supérieure à 10 m$^2$/g et un volume poreux total supérieur ou égal à 10 ml/100 g, le volume poreux correspondant aux pores de diamètre supérieur à 500 Å étant supérieur ou égal à 10 ml/100 g.

3. Procédé selon l'une des revendications 1 ou 2, **caractérisé en ce que** le catalyseur présente une surface spécifique supérieure à 50 m$^2$/g.

4. Procédé selon l'une des revendications 1 à 3, **caractérisé en ce que** le catalyseur présente un volume poreux total supérieur ou égal à 20 ml/100g avec un volume poreux correspondant aux pores de diamètre supérieur à 70 Å supérieur ou égal à 20 ml/100g.

5. Procédé selon l'une des revendications 1 à 3, **caractérisé en ce que** le catalyseur présente un volume poreux total supérieur ou égal à 15 ml/ 100g avec un volume poreux correspondant aux pores de diamètre supérieur à 200 Å supérieur ou égal à 15 ml/ 100g.

6. Procédé selon l'une des revendications précédentes , **caractérisé en ce que** les composés oxygénés supportés sur un support poreux sont des composés oxygénés d'éléments choisi dans le groupe comprenant le silicium, le titane, le zirconium, le vanadium, le niobium, le tantale, le tungstène, le molybdène, l'hafnium, le scandium, le phosphore, le bore, le fer, les alcalino-terreux, les terres rares ou leurs mélanges ou oxydes mixtes.

7. Procédé selon l'une des revendications précédentes, **caractérisé en ce que** le catalyseur comprend des anions choisis dans le groupe comprenant le fluor, des anions de formule générale (MxOy) dans la quelle M représente un élément choisi dans le groupe comprenant le silicium, l'arsenic, l'antimoine, l'azote, le soufre, le carbone et le phosphore, x étant un nombre entier compris entre 1 et 4 et y un nombre entier compris entre 1 et 8, ou des hétéropolyanions (HPA) de formule générale $X^{(n+)}T_{12}O_{40}^{(8-n)-}$ dans laquelle T signifie le tungstène ou le molybdène et X signifie le silicium, le germanium, le phosphore, l'arsenic ou le vanadium.

8.  Procédé selon l'une des revendications précédentes, **caractérisé en ce que** la concentration en composés oxygénés exprimés en poids d'élément par rapport au poids de catalyseur est comprise entre 1000 ppm et 30 %.

9.  Procédé selon la revendication 8, **caractérisé en ce que** la concentration en composés oxygénés est comprise entre 0,5% et 15% en poids par rapport au poids de catalyseur.

10. procédé selon la revendication 7, **caractérisé en ce que** la concentration en anions est comprise entre 0,5 et 15% en poids par rapport au poids de catalyseur.

11. Procédé selon l'une des revendications précédentes **caractérisé en ce que** le catalyseur particulaire est sous forme de billes, concassés, extrudés en forme de granulés cylindriques ou de forme multilobés creux ou pleins ou de nids d'abeille, pastilles.

12. Procédé selon l'une des revendications précédentes, **caractérisé en ce que** le support poreux est de l'alumine

13. Procédé selon l'une des revendications précédentes, **caractérisé en ce que** la réaction d'hydrolyse cyclisante est mise en oeuvre en phase vapeur.

**Patentansprüche**

1.  Verfahren zur cyclisierenden Hydrolyse einer Aminonitril-Verbindung zu Lactam durch Reaktion eines Aminonitrils der allgemeinen Formel (I)

$$N \equiv C - R - NH_2 \qquad\qquad (I)$$

in der

R einen substituierten oder unsubstituierten, aliphatischen, cycloaliphatischen oder arylaliphatischen Rest mit 3 bis 12 Kohlenstoffatomen darstellt, mit Wasser in Anwesenheit eines festen Katalysators, **dadurch gekennzeichnet, daß** der Katalysator ein Teilchenkatalysator ist, erhalten durch Ablagerung und/oder Adsorption von mindestens einer sauerstoffhaltigen Verbindung von mindestens einem Element, gewählt aus der Gruppe, die aus den Elementen gebildet wird, die aus den Gruppen 1 bis 16 des Periodensystems der Elemente (neue Klassifikation) stammen, wobei diese Liste ebenfalls die Seltenen Erden einschließt, auf einem Träger aus einem einfachen oder gemischten mineralischen Oxid oder einer Mischung von Oxiden von mindestens einem Element, gewählt aus der Gruppe, die gebildet wird durch Silicium, Aluminium, Titan, Zirkonium, Vanadium, Niob, Tantal, Wolfram, Molybdän, Eisen, die Seltenen Erden, und dadurch, daß er mindestens eine Makroporosität, die **gekennzeichnet ist durch** ein Porenvolumen, das Poren vom Durchmesser über 500 Å entspricht, von über oder gleich 5 ml/100 g umfaßt.

2.  Verfahren nach Anspruch 1, **dadurch gekennzeichnet, daß** der Teilchenkatalysator eine spezifische Oberfläche von über 10 m$^2$/g und ein Gesamtporenvolumen von über oder gleich 10 ml/100 g aufweist, wobei das Porenvolumen, das Poren vom Durchmesser von über 500 Å entspricht, über oder gleich 10 ml/100 g beträgt.

3.  Verfahren nach einem der Ansprüche 1 oder 2, **dadurch gekennzeichnet, daß** der Katalysator eine spezifische Oberfläche von über 50 m$^2$/g aufweist.

4.  Verfahren nach einem der Ansprüche 1 bis 3, **dadurch gekennzeichnet, daß** der Katalysator ein Gesamtporenvolumen von über oder gleich 20 ml/100 g aufweist, mit einem Porenvolumen, das Poren vom Durchmesser von über 70 Å entspricht, von über oder gleich 20 ml/100 g.

5.  Verfahren nach einem der Ansprüche 1 bis 3, **dadurch gekennzeichnet, daß** der Katalysator ein Gesamtporenvolumen von über oder gleich 15 ml/100 g aufweist, mit einem Porenvolumen, das Poren vom Durchmesser von über 200 Å entspricht, von über oder gleich 15 ml/100 g.

6.  Verfahren nach einem der vorstehenden Ansprüche, **dadurch gekennzeichnet, daß** die auf einen porösen Träger aufgebrachten sauerstoffhaltigen Verbindungen solche sauerstoffhaltigen Verbindungen von Elementen sind, die aus der Gruppe gewählt werden, die Silicium, Titan, Zirkonium, Vanadium, Niob, Tantal, Wolfram, Molybdän, Hafnium, Scandium, Phosphor, Bor, Eisen, die Erdalkalimetalle, die Seltenen Erden oder ihre Mischungen oder gemischten Oxide umfaßt.

7.  Verfahren nach einem der vorstehenden Ansprüche, **dadurch gekennzeichnet, daß** der Katalysator Anionen umfaßt, gewählt aus der Gruppe, die Fluor, die Anionen der allgemeinen Formel (MxOy), worin M ein Element darstellt, gewählt aus der Gruppe, die Silicium, Arsen, Antimon, Stickstoff, Schwefel, Kohlenstoff und Phosphor umfaßt, x eine ganze Zahl zwischen 1 und 4 ist und y eine ganze Zahl zwischen 1 und 8 ist, oder Heteropolyanionen (HPA) der allgemeinen Formel $X^{(n+)}T_{12}O_{40}^{(8-n)-}$ umfaßt, worin T Wolfram oder Molybdän und X Silicium, Germanium, Phosphor, Arsen oder Vanadium bezeichnen.

8.  Verfahren nach einem der vorstehenden Ansprüche, **dadurch gekennzeichnet, daß** die Konzentration an sauerstoffhaltigen Verbindungen, ausgedrückt in Gewicht Element, zwischen 1000 ppm und 30 % beträgt, bezogen auf das Gewicht des Katalysators.

9.  Verfahren nach Anspruch 8, **dadurch gekennzeichnet, daß** die Konzentration an sauerstoffhaltigen Verbindungen zwischen 0,5 Gew.-% und 15 Gew.-% beträgt, bezogen auf das Gewicht des Katalysators.

10. Verfahren nach Anspruch 7, **dadurch gekennzeichnet, daß** die Konzentration an Anionen zwischen 0,5 Gew.-% und 15 Gew.-% beträgt, bezogen auf das Gewicht des Katalysators.

11. Verfahren nach einem der vorstehenden Ansprüche, **dadurch gekennzeichnet, daß** der Teilchenkatalysator in Form von Kügelchen, Bruchstücken, Extrudaten in Form von zylindrischen Granulaten, oder in hohler oder voller multilobaler Form oder in Form von Bienenwaben oder Pastillen vorliegt.

12. Verfahren nach einem der vorstehenden Ansprüche, **dadurch gekennzeichnet, daß** der poröse Träger Aluminiumoxid ist.

13. Verfahren nach einem der vorstehenden Ansprüche, **dadurch gekennzeichnet, daß** die Reaktion der cyclisierenden Hydrolyse in der Dampfphase durchgeführt wird.

**Claims**

1.  Process for the cyclizing hydrolysis of an aminonitrile compound into a lactam by reaction of an aminonitrile of general formula (I):

$$N \equiv C - R - NH_2 \qquad\qquad (I)$$

in which R represents a substituted or unsubstituted aliphatic, cycloaliphatic or arylaliphatic radical comprising from 3 to 12 carbon atoms, with water, in the presence of a solid catalyst, **characterized in that** the catalyst is a particulate catalyst obtained by deposition and/or adsorption of at least one oxygenated compound of at least one element chosen from the group consisting of the elements belonging to groups 1 to 16 of the universal classification of the elements (new classification), this list also including rare-earth metals, on a support made of simple or mixed inorganic oxide or a mixture of oxides of at least one element chosen from the group consisting of silicon, aluminium, titanium, zirconium, vanadium, niobium, tantalum, tungsten, molybdenum, iron and rare-earth metals, and **in that** it comprises at least one macroporosity **characterized by** a pore volume, corresponding to pores greater than 500 Å in diameter, of greater than or equal to 5 ml/100 g.

2.  Process according to claim 1, **characterized in that** the particulate catalyst has a specific surface of greater than 10 m²/g and a total pore volume of greater than or equal to 10 ml/100 g, the pore volume corresponding to pores greater than 500 Å in diameter being greater than or equal to 10 ml/100 g.

3. Process according to either of claims 1 and 2, **characterized in that** the catalyst has a specific surface of greater than 50 m$^2$/g.

4. Process according to one of claims 1 to 3, **characterized in that** the catalyst has a total pore volume of greater than or equal to 20 ml/100 g with a pore volume corresponding to pores greater than 70 Å in diameter of greater than or equal to 20 ml/100 g.

5. Process according to one of claims 1 to 3, **characterized in that** the catalyst has a total pore volume of greater than or equal to 15 ml/100 g with a pore volume corresponding to pores greater than 200 Å in diameter of greater than or equal to 15 ml/100 g.

6. Process according to one of the preceding claims, **characterized in that** the oxygenated compounds supported on a porous support are oxygenated compounds of elements chosen from the group comprising silicon, titanium, zirconium, vanadium, niobium, tantalum, tungsten, molybdenum, hafnium, scandium, phosphorus, boron, iron, alkaline-earth metals and rare-earth metals or mixtures thereof or mixed oxides.

7. Process according to one of the preceding claims, **characterized in that** the catalyst comprises anions chosen from the group comprising fluorine, anions of general formula (MxOy) in which M represents an element chosen from the group comprising silicon, arsenic, antimony, nitrogen, sulphur, carbon and phosphorus, x being an integer between 1 and 4 and y being an integer between 1 and 8, or heteropolyanions (HPA) of general formula $X^{(n+)}T_{12}O_{40}^{(8-n)-}$ in which T is tungsten or molybdenum and X is silicon, germanium, phosphorus, arsenic or vanadium.

8. Process according to one of the preceding claims, **characterized in that** the concentration of oxygenated compounds, expressed by weight of element relative to the weight of catalyst, is between 1000 ppm and 30%.

9. Process according to claim 8, **characterized in that** the concentration of oxygenated compounds is between 0.5% and 15% by weight relative to the weight of catalyst.

10. Process according to claim 7, **characterized in that** the concentration of anions is between 0.5 and 15% by weight relative to the weight of catalyst.

11. Process according to one of the preceding claims, **characterized in that** the particulate catalyst is in the form of beads, crushings, extrudates in the form of cylindrical granules or in hollow or solid multilobe form or in the form of honeycombs or pellets.

12. Process according to one of the preceding claims, **characterized in that** the porous support is alumina.

13. Process according to one of the preceding claims, **characterized in that** the cyclizing hydrolysis reaction is carried out in vapour phase.